# EUROPÄISCHE PATENTSCHRIFT

(11) **EP 1 820 449 B1**
(45) Veröffentlichungstag und Bekanntmachung des Hinweises auf die Patenterteilung: **27.08.2008**
(21) Anmeldenummer: 07001742.1
(22) Anmeldetag: 21.02.2006
(51) Int. Cl.: A61B 6/00, A61B 6/12

(54) **Medizintechnisches Röntgendetektionssystem mit aktivem optischen Signalgeber**
Medical x-ray detection system with active optical signalling device
Système de détection par radiographie médicale doté d'un émetteur de signal optique actif

(43) Veröffentlichungstag der Anmeldung: 22.08.2007
(62) Teilanmeldung aus: 06003498.0
(73) Patentinhaber: BrainLAB AG, 85622 Feldkirchen (DE)
(72) Erfinder: Bogojevic, Aleksander, 81677 München (DE); Weiser, Manfred, Dr., 81673 München (DE)
(74) Vertreter: Schwabe - Sandmair - Marx

(56) Entgegenhaltungen:
- US-A1- 2005 242 289
- US-B1- 6 236 712
- US-B1- 6 470 207
- PATENT ABSTRACTS OF JAPAN Bd. 016, Nr. 264 (P-1370), 15. Juni 1992 (1992-06-15) & JP 04 065694 A (MATSUSHITA ELECTRIC IND CO LTD), 2. März 1992 (1992-03-02)

## Beschreibung

Die Erfindung betrifft eine medizintechnische Röntgendetektionseinrichtung mit einem Röntgendetektor, der das Vorhandensein einer Röntgenbestrahlung im medizinischen Umfeld erfasst, und einem Signalgeber, der ein Signal bezüglich des Vorhandenseins der Röntgenbestrahlung abgibt. Solche Röntgendetektionseinrichtungen werden in vielen Fällen dazu eingesetzt, bei der Registrierung von Röntgenbildern im Rahmen einer medizintechnischen Navigation genau festzustellen, wann (und damit in welcher Lage des Patienten) das Bild erstellt worden ist, um eine korrekte Zuordnung des Bildes im Rahmen der Navigation zu gestatten. Natürlich ist es grundsätzlich möglich, das Detektionssignal per Kabel zum Empfänger zu übertragen, z.B. zu einer weiterverarbeitenden Einheit (Navigationssystem). Solche Kabel müssen aber separat in der Röntgeneinrichtung bzw. der Registrierungseinrichtung verlegt werden und sie benötigen zusätzliche Schnittstellen an den das Signal empfangenden Komponenten, was diese Technik relativ aufwendig gestaltet.

Ein weiterer Ansatz im Stand der Technik besteht für den Anwendungsfall mit analogen Röntgenbild-Erzeugungssystemen darin, die Bildinformationen zu aktualisieren und bei Änderungen des Bildinhaltes eine Neuregistrierung vorzunehmen. Ein solches System ist beispielsweise aus der EP 1 260 179 B1 bekannt, jedoch sind noch viele ältere oder in spezieller Weise arbeitende Röntgengeräte im Gebrauch, deren Signal/Rauschen-Verhältnis ein solches Vorgehen schwierig macht oder nicht zulässt. Auch in Fällen, wo das Bild direkt am Röntgengerät manipuliert wird (z.B. skalliert, gedreht, gekippt oder im Kontrast verbessert) könnte dies zur Erfassung eines "neuen" Bildes führen, obwohl ein solcher Fall nicht vorliegt. Navigationsprobleme könnten entstehen.

Aus der US 6,470,207 B1 ist eine medizintechnische Röntgendetektionseinrichtung mit einem Röntgendetektor bekannt, der das Vorhandensein einer Röntgenstrahlung im medizinischen Umfeld erfasst. Ein Signalgeber des Röntgendetektionssystems gibt dabei ein Signal bezüglich des Vorhandenseins der Röntgenstrahlung an einen Rechner ab.

Aus der US 2005/0242289 A1 ist ein Dosimeter bekannt welches von einem Arzt am Körper getragen wird und eine LED aufweist, welche ein visuelles Signal abgibt, wenn eine bestimmte Strahlenbelastung überschritten wird.

Es ist die Aufgabe der vorliegenden Erfindung, eine medizinische Röntgendetektionseinrichtung bereitzustellen, welche die Probleme des Standes der Technik überwindet. Insbesondere soll das Vorhandensein einer Röntgenbestrahlung zuverlässig detektiert werden können, wobei das Detektionssignal in einfacher und kompatibler Weise weitergegeben werden kann.

Diese Aufgabe wird erfindungsgemäß durch eine medizintechnische Röntgendetektionseinrichtung gemäß dem Anspruch 1 gelöst. Die Unteransprüche beschreiben bevorzugte Ausführungsformen der Erfindung.

Gemäß der vorliegenden Erfindung umfasst der Signalgeber ein aktives, lichtabstrahlendes Signalmittel. Mit anderen Worten gibt der Signalgeber ein optisches Signal ab und hebt sich schon dadurch von einer relativ aufwändigen Kabelübertragung ab. Die aktiven Lichtabstrahlungen können unmittelbar und ohne Zeitverluste von einem Trackingsystem detektiert werden, so dass das Vorhandensein der Röntgenbestrahlung zeitlich sehr zuverlässig erfasst werden kann.

Das Signalmittel kann eine Leuchtdiode aufweisen, insbesondere kann es auch in Infrarotstrahler oder eine Infrarot-Leuchtdiode sein. Die Verwendung des Wellenbereiches des infraroten Lichtes zeichnet sich durch den Vorteil aus, dass Störungen durch Lichtblitze im Bereich des sichtbaren Lichtes keinen Einfluss auf die Signaldetektion haben. Es ist aber an dieser Stelle anzumerken, dass die Erfindung durchaus auch mit Signalmitteln funktionieren kann, die im Bereich des sichtbaren Lichtes abstrahlen. Es ist beispielsweise denkbar, das Signal eindeutig dadurch zu identifizieren, dass es eine bestimmte Signalabfolge umfasst. Wenn im Kontext der vorliegenden Beschreibung von der Abstrahlung von Licht gesprochen wird, umfasst dieser Begriff also sichtbare und/oder nicht sichtbare Anteile des elektromagnetischen Spektrums im Lichtbereich.

Die Energie für das Signalmittel kann in unterschiedlicher Weise bereitgestellt werden, und insbesondere umfasst die Erfindung eine eigene, dem Signalmittel zugeordnete Energieversorgung, insbesondere eine Batterie oder einen Akkumulator. Auch denkbar ist es, die Energie für das Signalmittel vom Röntgendetektor selbst (aus der Röntgenstrahlungsenergie) erzeugen zu lassen und direkt an das Signalmittel weiterzugeben. Hier wäre ein entsprechender Wandler einzusetzen.

Der Röntgendetektor kann im Rahmen der Erfindung den Beginn und/oder das Ende der Röntgenbestrahlung erfassen, wobei der Signalgeber entsprechende Signale abgibt.

Gemäß einem weiteren Aspekt der Erfindung betrifft diese ein medizintechnisches Röntgendetektionssystem mit einer Röntgendetektionseinrichtung wie sie beispielsweise oben in verschiedenen Ausführungen erläutert wurde, und mit einer Fluoroskop-Bildregistrierungseinheit, wobei Röntgendetektor und Signalgeber an der Fluoroskop-Bildregistrierungseinheit angeordnet sind. Es ist dabei gemäß einer Ausführungsform möglich, den Röntgendetektor im Strahlendurchgang der Fluoroskop-Bildregistrierungseinheit anzuordnen. Wichtig ist aber lediglich, dass der Röntgendetektor an einer Stelle angeordnet ist, wo er zuverlässig das Vorhandensein der Röntgenbestrahlung erfassen kann.

Der Signalgeber ist vorteilhafter Weise außen an der Fluoroskop-Bildregistrierungseinheit angeordnet, weil er dort sein Signal an von außen gut sichtbarer Stelle abgibt.

Es ist im Rahmen der Erfindung ebenfalls möglich, mehrere Signalgeber an der Fluoroskop-Bildregistrierungseinheit anzuordnen, ebenso können mehrere Röntgendetektoren dort angeordnet sein. Hierdurch lassen sich eine Redundanz und auch eine Ausfallsicherheit herstellen. Bei einer Ausführungsvariante trägt die Fluoroskop-Bildregistrierungseinheit beispielsweise drei Signalgeber und auch drei Röntgendetektoren, die in jedweder möglichen Weise einander zugeordnet sein können. Grundsätzlich sind auch solche Fälle denkbar, wo ein einziger Röntgendetektor mehrere Signalgeber ansteuert oder ein Signalgeber von mehreren Röntgendetektoren angesteuert wird.

Die Erfindung umfasst in einer vorteilhaften Ausführung auch nachrüstbare oder zurüstbare Röntgendetektionseinrichtungen, und dabei besteht die Möglichkeit, Signalgeber und/oder Röntgendetektor (EN) zusätzlich anbringbar und wieder abnehmbar an der Fluoroskop-Bildregistrierungseinheit vorzusehen.

Einem weiteren Aspekt der vorliegenden Erfindung gemäß wird ein medizintechnisches Röntgendetektionssystem bereitgestellt, das eine Röntgendetektionseinrichtung aufweist, wie sie oben beschrieben wurde, und zusätzlich ein optisches medizintechnisches Trackingsystem, insbesondere ein Infrarot-Trackingsystem, wobei der Signalgeber im Erfassungsbereich des Trackingsystems angeordnet ist und das Signal des Signalgebers vom Trackingsystem erfasst wird. In diesem Umfeld zeigt die Erfindung besonders ihre Vorteile, da sich ein optischer Signalgeber problemlos und unmittelbar in eine solche Trackingsystem-Umgebung einordnen lässt. Weil in vielen Navigations-Umfeldern solche optischen Trackingsysteme vorhanden sind oder verwendet werden, lässt sich das Detektionssignal problemlos erfassen, verarbeiten und in den Ablauf eingliedern. Natürlich kann ein solches System (mit Trackingsystem) auch die Merkmale aufweisen, die oben für das medizintechnische Röntgendetektionssystem beschrieben wurden.

Bei einer Ausführungsform der erfindungsgemäßen Röntgendetektion, deren verfahrensmäßige Merkmale natürlich insgesamt zur vorliegenden Offenbarung gehören, werden also Röntgenstrahlen, die während der Behandlung oder bei der Behandlungsvorbereitung erzeugt werden, durch eine kleine Detektoreinheit erfasst. Die Information über den Beginn und das Ende der Bestrahlungszeit wird durch eine aktive Komponente weitergegeben, welche speziell auch innerhalb einer Anordnung passiver Marker eingebettet sein kann. Die Integration passiver und aktiver Lichtübertragung führt hier zur gegenseitigen Ergänzung; beide Signale sind kabellos übertragbar und erfassbar. Während für die Ortung eines Registrierungs-Kits durchaus passive Marker an einem solchen Kit ausreichend sind, ergänzt die aktive Signalübertragung für die Röntgendetektion diese Informationsübertragung in optimaler Weise. Es ist somit möglich, Auslösesignale für den Beginn oder das Ende der Bestrahlung beispielsweise in ein bekanntes Navigationsumfeld einzugliedern, das auf Reflektionsmarker-Technologie basiert. Die Integration eines oder mehrerer Röntgendetektoren in das Fluoroskop-Bildregistrierungskit und die Ergänzung der Markeranordnung des Kits durch den erfindungsgemäßen Signalgeber gestattet somit eine verbesserte Erfassung neu akquirierter Bilder, die eine hohe Zuverlässigkeit mit sich bringt. Durch die erfindungsgemäße Steuerung bzw. Kontrolle über diesen kritischen Teil der Fluoroskop-Bildakquirierung werden alle Röntgen-Tracking- oder Navigationssysteme von stark reduzierten Trackingfehlern profitieren. Dies gilt speziell für C-Bogen-Röntgensysteme mit analoger Bildübertragung (Video), weil der erfindungsgemäße Ansatz nicht die Nachteile rein Software basierter Bildvergleichsmethoden aufweist.

Im Umfeld eines Infrarot-Trackingsystems bzw. eines hieran angeschlossenen Navigationssystems ist die vorliegende Erfindung besonders wertvoll, weil sie gerade solche vorhandenen Systeme optimal ausnutzt, die passive Markeranordnungen und Infrarotkameras umfassen. Eine zusätzliche aktive Infrarotkomponente (Signalgeber) würde automatisch durch das Kamerasystem erfasst, wobei die Wellenlänge des Infrarotlichts natürlich im Erfassungsbereich des Kamerasystems liegen muss, und somit wäre ihre Position für die weitere Auswertung verfügbar. Eine solche Eingliederung kann einfach durch wenige Änderungen in der Software umgesetzt werden, die für die Erfassung anderer Behandlungseinrichtungen bzw. behandlungsuntertützender Einrichtungen mit Positionsmarkern verantwortlich ist, und die Anwendung selbst könnte entsprechende Algorithmen implementieren. An der existierenden Hardware müssen keine Änderungen durchgeführt werden.

Speziell für C-Bogen-Fluoroskopiegeräte mit analogem Übertragungsmodus bietet die vorliegende Erfindung mit der automatischen Signaldetektion Vorteile. Es kann beispielsweise die Information durch den Bildvergleich durch das Auslösesignal des Signalgebers bei der Erfassung der Röntgenbestrahlung ergänzt werden. Der exakte Zeitpunkt zum Speichern der Trackinginformation würde dann aus den im Koinzidenz-Kriterium ermittelt, gemäß dem beide Vorgänge zur selben Zeit stattfinden müssen, und zwar innerhalb eines sehr kleinen Zeitintervalls. Eine solchermaßen zuverlässigere Möglichkeit, korrekte Trackinginformationen zu erhalten, wird auch die Sicherheit der Patienten erhöhen.

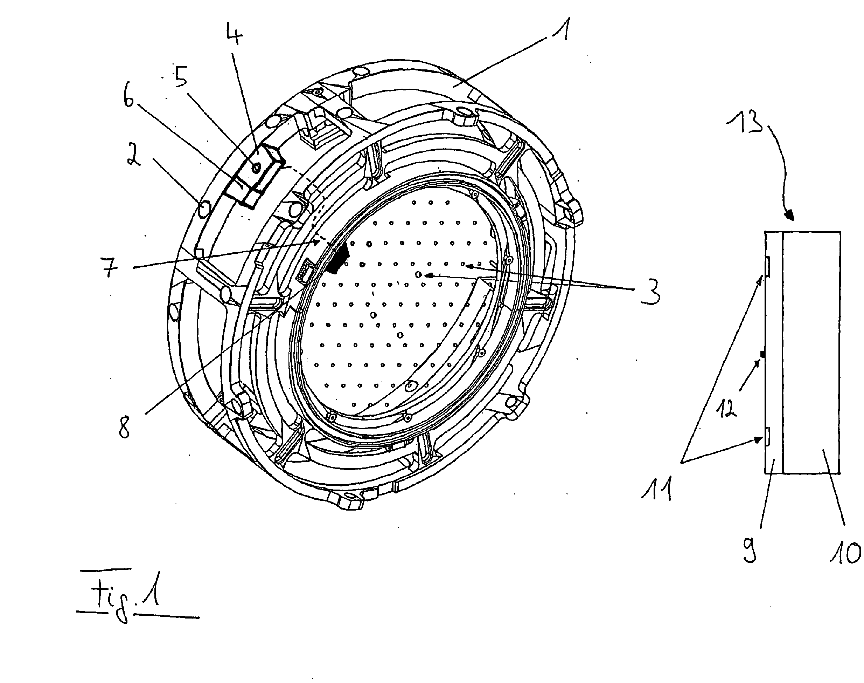

Die Erfindung wird im Weiteren anhand einer Ausführungsform und unter Bezugnahme auf die beiliegende Zeichnung näher erläutert. Sie kann alle hierin aufgeführten Merkmale in jedweder Kombination umfassen. Die einzige beiliegende Figur 1 zeigt ein Fluoroskop-Registrierungskit, das eine Röntgendetektionseinrichtung gemäß der vorliegenden Erfindung umfasst.

Das in der Figur dargestellte Registrierungskit 1 ist ein kreisförmiger Aufsatz für den Bildverstärker eines C-Bogen-Röntgengerätes (Fluoroskop). Das Kit 1 umfasst im Strahlendurchgang einen Einsatz mit Wolframkugeln 3 für die interne Registrierung der Projektionseigenschaften sowie mehrere in spezieller Anordnung vorgesehene passive Reflektionsmarker 2, die für das Tracking des Kits 1 in einem Tracking- und Navigationsumfeld vorgesehen sind. Rechts in Figur 1 ist nur schematisch eine Tracking- bzw. Navigationsanordnung aufgezeigt. Das System 13 besteht aus dem Trackingsystem 9 und dem Navigationssystem 10, welches die Trackinginformationen (räumliche Ortung, Bestimmung der Behandlungsgeräte und der behandlungsunterstützenden Geräte, insbesondere räumliche Ortung, Bestimmung des Registrierungskits 1) verarbeitet und auch ausgeben kann. Das Trackingsystem 9 weist zwei Kameras 11 auf, sowie einen Infrarot-Abstrahler 12. Infrarotlicht vom Abstrahler 12 wird beispielsweise durch die Reflektionsmarker 2 am Kit 1 reflektiert und von den Kameras 11 empfangen. Aus diesen Empfangssignalen errechnet das Trackingsystem 9 dann die räumliche Lage des Kits 1 und gibt diese zur Verarbeitung an das Navigationssystem 10 weiter.

Am Registrierungskit 1 ist am Innenumfang im Strahlendurchgang ein kleiner Röntgendetektor 8 befestigt. Die Befestigung erfolgt an einer Stelle im Außenbereich der Bildverstärkungsebene, die nahe am Registrierungskit 1 liegt, und es ist zu beachten, dass der Detektor die Befestigung des Kits nicht stört. Diese Position gestattet die Erfassung von Röntgenstrahlen, weil diese in Form eines Kegels aufgebracht wird, aber sie stört den Bildinhalt nicht wesentlich.

Auf der gegenüberliegenden Seite des Registrierungskits, und zwar außen am unteren überstehenden Rand ist die aktive optische Komponente fixiert, nämlich der Signalgeber 4 mit dem aktiven, lichtabstrahlenden Signalmittel 5, das im vorliegenden Fall eine Infrarot-LED ist. In der hier dargestellten, vorteilhaften Ausführungsform liegt die LED 5 in einer speziellen im Navigationssystem bekannten Position in Bezug auf das Referenzsystem der Anordnung der Reflektionsmarker 2. Zusätzlich ist eine unabhängige Energieversorgung vorgesehen, nämlich eine Batterie 6 (bzw. Akkumulator), und der Detektor 8 ist mit der Einheit aus Signalgeber 4 und Batterie 6 über die Leitung 7 verbunden. Die Batterie kann für den Signalgeber 4 aber auch für den Detektor 8 die notwendige Energie liefern.

Wenn ein neues Fluoroskopiebild akquiriert wird, wird Strahlung aus einer Röntgenquelle in konischer Form auf einen Bildverstärker emittiert. Weil der Detektor 8 an einer Position befestigt ist, die durch die Befestigung des Kits 1 nicht gestört wird, kann er eine solche Strahlung erfassen und ein Signal auslösen, welches durch den Signalgeber 4, d.h. die LED 5 emittiert wird. Wenn die Strahlung unter einen bestimmten Schwellwert fällt, wird das Auslösesignal gestoppt. Durch die geeignete Auswahl des Schwellwertes kann eine verlässliche Messung für die Start- und Endzeit der emittierten Strahlung durchgeführt werden, wodurch Fehlauslösungen vermieden werden, die von Hintergrundstrahlung herrühren.

Das optische Auslösesignal wird im Infrarot-Wellenlängenbereich von der LED 5 emittiert, und es ist deshalb für die Kameras 11 sichtbar, die zur Navigation verwendet werden. Weil die Position der LED 5 im Verhältnis zu der Anordnung der passiven Marker 2 systembekannt ist (Navigationssystem) stellt die gleichzeitige Beobachtung der Markeranordnung und der LED sicher, dass keine zufällige Reflektion fälschlich als Auslösesignal interpretiert wird.

Im Navigationssystem 10 werden die Trackingdaten der Kameras 11 bzw. des Trackingsystems 9 permanent aktualisiert und analysiert. Wenn die Markergeometrie des Registrierungskits 1 beobachtet wird, kann ein zusätzlicher Test für das Vorhandensein des Signals von der LED 5 durchgeführt werden, und zwar in einfacher Weise dadurch, dass geprüft wird, ob das Signal einem zusätzlichen Marker in einer speziellen Anordnung gegenüber den Referenzrahmen des Registrierungskits 1 entspricht.

Für die Entscheidung, ob ein neues Bild akquiriert worden ist, sind zwei Modi denkbar:

Einerseits kann die Zeit, zu der das Signal zuerst erfasst wird, als Startzeit der Bestrahlung registriert werden, und die Zeit, zu der das Signal nicht mehr beobachtbar ist, wird als Endzeit registriert. Es wird keine weitere Kontrolle durchgeführt und die Akquirierung eines neuen Bildes wird dem System signalisiert.

Eine zweite Möglichkeit besteht darin, das Auslösesignal mit einem anderen Signal zu korrelieren, das aus dem Bildvergleich stammt, der ohnehin im Hintergrund durchgeführt wird. Ein neues Bild wird erkannt, und nur dann erkannt, wenn sowohl das Auslösesignal als auch das positive Resultat des Bildvergleichs in zeitlicher Übereinstimmung sind (mit nur sehr geringer zusätzlicher Toleranz).

## Patentansprüche

1. Medizintechnisches Röntgendetektionssystem mit einer medizintechnischen Röntgendetektionseinrichtung, die einen Röntgendetektor (8), der das Vorhandensein einer Röntgenbestrahlung im medizinischen Umfeld erfasst, und einen Signalgeber (4), der ein Signal bezüglich des Vorhandenseins der Röntgenbestrahlung abgibt, aufweist ; und mit ein em optischen medizintechnischen Trackingsystem (13), insbesondere ein Infrarot-Trackingsystem, wobei der Signalgeber (4) im Erfassungsbereich des Trackingsystems (13) angeordnet ist, **dadurch gekennzeichnet dass**, der Signalgeber (4) ein aktives, Licht abstrahlendes Signalmittel (5) umfasst und das Trackingsystem (13) das Signal des Signalgebers (4) erfasst.

2. Medizintechnisches Röntgendetektionssystem nach Anspruch 1, **dadurch gekennzeichnet, dass** das Signalmittel eine Leuchtdiode (5) aufweist.

3. Medizintechnisches Röntgendetektionssystem nach Anspruch 1 oder 2, **dadurch gekennzeichnet, dass** das Signalmittel einen Infrarotstrahler aufweist, insbesondere eine Infrarotlicht-Leuchtdiode (5).

4. Medizintechnisches Röntgendetektionssystem nach einem der Ansprüche 1 bis 3, **dadurch gekennzeichnet, dass** das Signalmittel an eine eigene zugeordnete Energieversorgung angeschlossen ist, insbesondere an eine Batterie (6) oder einen Akkumulator.

5. Medizintechnisches Röntgendetektionssystem nach einem der Ansprüche 1 bis 4, **dadurch gekennzeichnet, dass** der Röntgendetektor den Beginn und/oder das Ende der Röntgenbestrahlung erfasst und der Signalgeber entsprechende Signale abgibt.

6. Medizintechnisches Röntgendetektionssystem nach einem der Ansprüche 1 bis 5, und mit einer Fluoroskop-Bildregistrierungseinheit (1), wobei Röntgendetektor (8) und Signalgeber (4) an der Fluoroskop-Bildregistrierungseinheit (1) angeordnet sind.

7. Medizintechnisches Röntgendetektionssystem nach Anspruch 6, **dadurch gekennzeichnet, dass** der Röntgendetektor (8) im Strahlendurchgang der Fluoroskop-Bildregistrierungseinheit (1) angeordnet ist.

8. Medizintechnisches Röntgendetektionssystem nach Anspruch 6 oder 7, **dadurch gekennzeichnet, dass** der Signalgeber (4) außen an der Fluoroskop-Bildregistrierungseinheit (1) angeordnet ist.

9. Medizintechnisches Röntgendetektionssystem nach einem der Ansprüche 6 bis 8, **dadurch gekennzeichnet, dass** mehrere Signalgeber (4) an der Fluoroskop-Bildregistrierungseinheit (1) angeordnet sind.

10. Medizintechnisches Röntgendetektionssystem nach einem der Ansprüche 6 bis 9, **dadurch gekennzeichnet, dass** mehrere Röntgendetektoren (8) an der Fluoroskop-Bildregistrierungseinheit (1) angeordnet sind.

11. Medizintechnisches Röntgendetektionssystem nach einem der Ansprüche 6 bis 10, **dadurch gekennzeichnet, dass** Signalgeber (4) und/oder Röntgendetektor(en) zusätzlich anbringbar und wieder abnehmbar an der Fluoroskop-Bildregistrierungseinheit (1) vorgesehen sind.

## Claims

1. A medical x-ray detection system comprising a medical x-ray detection device comprising an x-ray detector (8) which detects the presence of x-ray radiation in the medical environment, and a signal emitter (4) which emits a signal with respect to the presence of the x-ray radiation; and comprising an optical medical tracking system (13), in particular an infrared tracking system, wherein the signal emitter (4) is arranged within the detection range of the tracking system (13); **characterised in that** the signal emitter (4) includes an active light-emitting signal means (5) and the tracking system (13) detects the signal of the signal emitter (4).

2. The medical x-ray detection system according to claim 1, **characterised in that** the signal means comprises an LED (5).

3. The medical x-ray detection system according to claim 1 or 2, **characterised in that** the signal means comprises an infrared emitter, in particular an infrared LED (5).

4. The medical x-ray detection system according to any one of claims 1 to 3, **characterised in that** the signal means is connected to an assigned power supply of its own, in particular a battery (6) or rechargeable battery.

5. The medical x-ray detection system according to any one of claims 1 to 4, **characterised in that** the x-ray detector detects the start and/or end of the x-ray radiation and the signal emitter emits corresponding signals.

6. The medical x-ray detection system according to any one of claims 1 to 5, and comprising a fluoroscopic image registration unit (1), wherein the x-ray detector (8) and the signal emitter (4) are arranged on the fluoroscopic image registration unit (1).

7. The medical x-ray detection system according to claim 6, **characterised in that** the x-ray detector (8) is arranged in the radiation transmission path of the fluoroscopic image registration unit (1).

8. The medical x-ray detection system according to claim 6 or 7, **characterised in that** the signal emitter (4) is arranged on the outside of the fluoroscopic image registration unit (1).

9. The medical x-ray detection system according to any one of claims 6 to 8, **characterised in that** several signal emitters (4) are arranged on the fluoroscopic image registration unit (1).

10. The medical x-ray detection system according to any one of claims 6 to 9, **characterised in that** several x-ray detectors (8) are arranged on the fluoroscopic image registration unit (1).

11. The medical x-ray detection system according to any one of claims 6 to 10, **characterised in that** a signal emitter (4) or signal emitters (4) and/or an x-ray detector or x-ray detectors are additionally provided on the fluoroscopic image registration unit (1) such that they are attachable and re-detachable.

## Revendications

1. Système médical de détection radiographique comportant un dispositif médical de détection radiographique qui comporte un détecteur radiographique (8), lequel relève la présence d'un rayonnement X dans l'environnement médical, ainsi qu'un générateur de signaux (4) qui délivre un signal relatif à la présence du rayonnement X, et avec un système optique médical de poursuite (13), en particulier un système de poursuite à infrarouge, le générateur de signaux (4) étant disposé dans la zone de détection du système de poursuite (13), **caractérisé en ce que** le générateur de signaux (4) comprend un moyen de signalisation (5) actif, émettant de la lumière, et le système de poursuite (13) détecte le signal du générateur de signaux (14).

2. Système médical de détection radiographique selon la revendication 1, **caractérisé en ce que** le moyen de signalisation comporte une diode luminescente (5).

3. Système médical de détection radiographique selon la revendication 1 ou 2, **caractérisé en ce que** le moyen de signalisation comporte un émetteur infrarouge, en particulier une diode luminescente à lumière infrarouge (5).

4. Système médical de détection radiographique selon l'une quelconque des revendications 1 à 3, **caractérisé en ce que** le moyen de signalisation est raccordé à sa propre alimentation en énergie associée, en particulier à une batterie (6) ou à un accumulateur.

5. Système médical de détection radiographique selon l'une quelconque des revendications 1 à 4, **caractérisé en ce que** le détecteur radiographique relève le début et/ou la fin du rayonnement X et le générateur de signaux délivre des signaux correspondants.

6. Système médical de détection radiographique selon l'une quelconque des revendications 1 à 5, et avec une unité d'enregistrement d'image fluoroscopique (1), le détecteur radiographique et le générateur de signaux (4) étant disposés sur l'unité d'enregistrement d'image fluoroscopique (1).

7. Système médical de détection radiographique selon la revendication 6, **caractérisé en ce que** le détecteur radiographique (8) est disposé dans le parcours des rayons de l'unité d'enregistrement d'image fluoroscopique (1).

8. Système médical de détection radiographique selon la revendication 6 ou 7, **caractérisé en ce que** le générateur de signaux (4) est disposé à l'extérieur sur l'unité d'enregistrement d'image fluoroscopique (1).

9. Système médical de détection radiographique selon l'une quelconque des revendications 6 à 8, **caractérisé en ce que** plusieurs générateurs de signaux (4) sont disposés sur l'unité d'enregistrement d'image fluoroscopique (1).

10. Système médical de détection radiographique selon l'une quelconque des revendications 6 à 9, **caractérisé en ce que** plusieurs détecteurs radiographiques (8) sont disposés sur l'unité d'enregistrement d'image fluoroscopique (1).

11. Système médical de détection radiographique selon l'une quelconque des revendications 6 à 10, **caractérisé en ce que** le générateur de signaux (4) et/ou le(les) détecteur(s) radiographique(s) sont prévus en outre de manière à pouvoir être placés puis retirés de l'unité d'enregistrement d'image fluoroscopique (1).
